Europäisches Patentamt

European Patent Office

Office européen des brevets

(19)

(11) Veröffentlichungsnummer: **0 257 519**
**B1**

(12) **EUROPÄISCHE PATENTSCHRIFT**

(45) Veröffentlichungstag der Patentschrift:
08.11.89

(51) Int. Cl.⁴: **C07C 69/34, C07C 69/44,**
**C07C 67/48, C07C 67/38**

(21) Anmeldenummer: **87111933.5**

(22) Anmeldetag: **18.08.87**

(54) Verfahren zur Behandlung von wässrigen Lösungen, die bei der Carbalkoxylierung von olefinsch ungesättigten Verbindungen anfallen.

(30) Priorität: **21.08.86 DE 3628357**

(43) Veröffentlichungstag der Anmeldung:
**02.03.88 Patentblatt 88/9**

(45) Bekanntmachung des Hinweises auf die Patenterteilung:
**08.11.89 Patentblatt 89/45**

(84) Benannte Vertragsstaaten:
**BE DE ES FR GB IT NL**

(56) Entgegenhaltungen:
**EP-A- 0 008 412**
**EP-A- 0 080 957**

**PATENT ABSTRACTS OF JAPAN; unexamined applications, C Field, Band 5, Nr. 12, 24. Jänner 1981 THE PATENT OFFICE JAPANESE GOVERNMENT Seite 139 C 40**

(73) Patentinhaber: **BASF Aktiengesellschaft,**
**Carl-Bosch-Strasse 38, D-6700 Ludwigshafen(DE)**

(72) Erfinder: **Bertleff, Werner, Dr., Neuzenlache 3,**
**D-6806 Viernheim(DE)**
Erfinder: **Maerkl, Robert, Dr., Schulstrasse 17,**
**D-6701 Fussgoenheim(DE)**
Erfinder: **Magnussen, Peter, Dr.,**
**Professor-Dillinger-Weg 25, D-6702 Bad Duerkheim(DE)**
Erfinder: **Kuehn, Gebhard, Am Weidenschlag 92,**
**D-6700 Ludwigshafen(DE)**
Erfinder: **Stops, Peter, Limburgstrasse 12,**
**D-6701 Altrip(DE)**

## Beschreibung

Gegenstand der Erfindung ist ein Verfahren zur Behandlung von wäßrigen Lösungen, die bei der Carbalkoxylierung von olefinisch ungesättigten Verbindungen anfallen und die heterocyclische aromatische Stickstoffbasen, deren Umwandlungsprodukte sowie niedere Fettsäuren und/oder deren Salze enthalten.

Bei der Carbalkoxylierung von olefinisch ungesättigten Verbindungen in Gegenwart von heterocyclischen aromatischen Stickstoffbasen fallen im Verlaufe des Verfahrens behandlungsbedürftige wäßrige Lösungenen an die solche heterocyclische aromatische Stickstoffbasen und deren Umwandlungsprodukte enthalten. Aus der europäischen Patentanmeldung 0 008 412 ist ein Verfahren bekannt, bei dem man wäßrige Lösungen, die heterocyclische aromatische Stickstoffbasen sowie deren Umwandlungsprodukte enthalten die bei der Katalysatorherstellung anfallen, bei einer Temperatur von 250 bis 300°C behandelt. Dieses Verfahren hat den Nachteil, daß noch erhebliche Mengen an Umwandlungsprodukten von heterocyclischen aromatischen Stickstoffbasen in der wäßrigen Lösung verbleiben und zudem, falls Kobaltsalze in der wäßrigen Lösung enthalten sind, metallisches Kobalt abgeschieden wird.

Es war deshalb die technische Aufgabe gestellt, ein Verfahren zur Behandlung von wäßrigen Lösungen, die heterocyclische aromatische tert. Stickstoffbasen und deren Umwandlungsprodukte enthalten, zur Verfügung zu stellen, bei dem die Rückgewinnung an heterocyclischen aromatischen Stickstoffbasen erhöht wird und zudem falls Kobaltsalze zugegen sind, die Abscheidung von metallischem Kobalt vermieden wird.

Diese technische Aufgabe wird gelöst in einem Verfahren zur Behandlung von wäßrigen Lösungen, die bei der Carbalkoxylierung von olefinisch ungesättigten Verbindungen anfallen und die heterocyclische aromatische Stickstoffbasen, deren Umwandlungsprodukte sowie niedere Fettsäuren und/oder deren Salze enthalten, wobei man

a) die wäßrige Lösung unter einem Druck von 10 bis 300 bar auf eine Temperatur von 150 bis 300 °C erhitzt und

b) anschließend die tertiären aromatischen Stickstoffbasen, die aus den Umwandlungsprodukten zurückgebildet wurden, abtrennt.

Ein Vorteil des neuen Verfahrens kann darin liegen, vorhandene heterocyclische aromatische Stickstoffverbindungen vor der Druck- und Temperaturbehandlung abzutrennen.

Das neue Verfahren hat den Vorteil, daß es einfach durchführbar ist und keines großen technischen Aufwandes bedarf. Weiter hat das neue Verfahren den Vorteil, daß die Ausbeute an heterocyclischen aromatischen Stickstoffbasen, die zurückgewonnen werden, erhöht wird. Ferner hat das neue Verfahren den Vorteil, daß die Abscheidung von metallischem Kobalt vermieden wird, falls Kobaltsalze in der wäßrigen Lösong enthalten sind. Sofern Kobaltsalze enthaltende Lösungen vorliegen, hat das neue Verfahren den Vorteil, daß diese Lösungen problemlos für die Herstellung von Katalysatoren für die Carbalkoxylierung verwendet können, ohne daß es im Laufe des Verfahrens zu unerwünschten Abscheidungen kommt.

Entsprechend der Erfindung geht man von wäßrigen Lösungen aus, die heterocyclische aromatische tert. Stickstoffbasen, deren Umwandlungsprodukte sowie niedere Fettsäuren und/oder deren Salze enthalten. Bevorzugte heterocyclische aromatische Stickstoffbasen sind Pyridin, Chinolin oder Isochinolin, die jeweils 1 bis 2 Alkylreste mit 1 bis 10 Kohlenstoffatomen, Cycloalkylreste mit 5 bis 8 Kohlenstoffatomen, Aralkylreste mit 7 bis 8 Kohlenstoffatomen oder Arylreste mit 6 bis 10 Kohlenstoffatomen z.B. Phenylreste als Substitutenten haben können. Bevorzugte Substituenten sind Alkylreste mit der genannten Kohlenstoffzahl. Insbesondere enthalten die wäßrigen Lösungen Pyridin, Alkyl- oder Dialkylpyridine mit $C_1$- bis $C_4$-Alkylresten. In der Regel sind solche heterocyclischen aromatischen Stickstoffbasen in einer Menge von 1 bis 10 Gew.-% in der wäßrigen Lösung vorhanden.

Unter Umwandlungsprodukten von heterocyclischen aromatischen Stickstoffbasen seien erfindungsgemäß, die sich aus solchen Stickstoffbasen während der Carbalkoxylierung bildenden Verbindungen, verstanden. Es handelt sich um eine Vielzahl von Stickstoffverbindungen, deren Zusammensetzung nicht näher bekannt ist. Bei der Verwendung von Pyridin als Stickstoffverbindung, bilden sich beispielsweise N-Methylpyridiniumsalze, N-Methyldihydropyridin sowie höhermolekulare Stickstoffbasen und andere Amine. Solche Umwandlungsprodukte von heterocyclischen aromatischen Stickstoffbasen sind in der wäßrigen Lösung im allgemeinen in einer Menge von 1 bis 10.-% enthalten.

Bevorzugte niedere Fettsäuren haben 1 bis 4 Kohlenstoffatome, insbesondere 2 oder 3 Kohlenstoffatome geeignete Fettsäuren sind z.B. Essigsäure, Propionsäure oder Buttersäure. In der Regel enthält die wäßrige Lösung Essigsäure. Die Fettsäuren sind im allgemeinen in einer Konzentration von 3 bis 20 Gew.-% enthalten.

Bevorzugte Salze von niederen Fettsäuren sind Alkalisalze, insbesondere Kalium- oder Natriumsalze. In der Regel handelt es sich um Kalium- oder Natriumsalze der obengenannten Fettsäuren, insbesondere Acetate. Solche Salze sind im allgemeinen in einer Menge von 5 bis 25 Gew.-% enthalten.

Darüber hinaus können die wäßrigen Lösungen Kobaltsalze der vorgenannten niederen Fettsäuren, insbesondere Kobaltacetat enthalten. Im allgemeinen beträgt der Gehalt an Kobaltsalzen von 0,1 bis 10 Gew.-%.

Eine typische Ausgangslösung enthält beispielsweise 3 bis 6 Gew.-%, heterocyclische aromatische tert. Stickstoffbasen, 1 bis 8 Gew.-% von deren Umwandlungsprodukten, 5 bis 10 Gew.-% Essigsäure und 3 bis 8 Gew.-% Kobaltacetat. Eine andere typische Ausgangslösung enthält beispielsweise 0,1 bis 1 Gew.-% heterocyclische aromatische tert. Stickstoffbasen, 3 bis 10 Gew.-% von deren Umwandlungsprodukten sowie 10 bis 15 Gew.-% Natriumacetat. Solche Lösungen fallen beispielsweise an bei der oxidativen Entkobaltung nach der Carbalkoxylierung, nach der Extraktion von Kobaltcarbonylkatalysator, sofern Kobaltsalzlösungen aus der oxidativen Entkobaltung für die Katalysatorherstellung zurückgeführt werden oder aus solchen Lösungen Kobalt, z.B. mit Natriumcarbonat, ausgefällt und abgetrennt wurde.

Vorteilhaft wird von den behandlungsbedürftigen wäßrigen Lösungen zunächst der freie Anteil an heterocyclischen aromatischen tert. Stickstoffbasen abgetrennt. Dies erfolgt z.B. durch Strippen mit den Inertgasen oder mit Dampf, insbesondere durch Destillation als Azeotrop mit Wasser.

Diese Abtrennung ist jedoch nicht zwingend, da das Vorhandensein von heterocyclischen aromatischen Stickstoffbasen in geringen Konzentrationen von etwa 1 bis 5 Gew.-% die weiteren Schritte nicht beeinflußt.

Anschließend wird die verbleibende wäßrige Lösung unter einem Druck von 10 bis 300 bar, insbesondere 10 bis 50 bar, zweckmäßig unter dem sich einstellenden Eigendruck auf eine Temperatur von 150 bis 300°C, insbesondere 150 bis 240°C, vorzugsweise eine Temperatur von 160 bis 240°C, erhitzt. Die Behandlungsdauer beträgt in der Regel von 15 bis 240 min..

Es hat sich bewährt, wenn man die Behandlung in Gegenwart von Oxidationsmitteln durchführt. Geeignete Oxidationsmittel sind beispielsweise Wasserstoffperoxid, molekularer Sauerstoff oder solchen enthaltende Gase wie Luft. Die Mitverwendung von Oxidationsmitteln hat sich insbesondere bei der Behandlung von Kobaltsalzen enthaltenden wäßrigen Lösungen bewährt.

Nach der thermischen Behandlung werden die nunmehr aus den Umwandlungsprodukten zurückgebildeten heterocyclischen aromatischen tert. Stickstoffbasen abgetrennt z.B. durch Strippen mit Inertgasen oder Dampf, insbesondere durch Destillation als Azeotrop mit Wasser. Zweckmäßig werden die Azeotrope von heterocyclischen aromatischen tert. Stickstoffbasen mit Wasser gemeinsam aufgearbeitet und z.B. durch Destillation mit Schleppmitteln Wasser entfernt und reine Stickstoffbasen erhalten.

Das Verfahren nach der Erfindung sei an folgenden Beispielen veranschaulicht.

Beispiel 1

In einem Hochdruckgefäß wurden 3 Mol 3-Pentensäuremethylester, 0,6 Mol der nachfolgend genannten Stickstoffbasen, 3,75 Mol Methanol und 0,13 Mol Kobalt als Kobaltoctacarbonyl vorgelegt. Die Umsetzung wurde mit Kohenmonoxid bei 165°C und 280 bar über 20 Stunden durchgeführt. Nach dem Abkühlen und Entspannen wurde der Austrag mit 300 ml Wasser und 50 g Essigsäure versetzt und unter Einleiten von Luft 1 Stunde am Rückfluß erhitzt. Nach dem Abkühlen wurde mit 300 ml Cyclohexan extrahiert. Aus der so erhaltenen wäßrigen Phase wurde der noch vorhandene Anteil an eingesetzter Stickstoffbase als Azeotrop mit Wasser vollständig abdestilliert. Der verbleibende Sumpf wurde in einem Hochdruckgefäß 4 Stunden unter autogenem Druck auf 250°C erhitzt. Nach Abkühlen und Entspannen wurde die rückgebildete Stickstoffbase abdestilliert und bestimmt. Die Ergebnisse für die einzelnen Stickstoffbasen sind aus der folgenden Tabelle zu entnehmen.

Tabelle 1

| N-Base | % Verlust nach Carbalkoxylierung | Neubildung g nach therm. Behandlung | % Rückgewinnung |
|--------|----------------------------------|-------------------------------------|-----------------|
| Pyridin | 24,4 | 6,14 | 52,4 |
| Beta-Picolin | 27,8 | 6,25 | 40,3 |
| Gamma-Picolin | 28,5 | 4,77 | 30,0 |

Beispiel 2

Man verfährt wie in Beispiel 1 beschrieben, verwendet Beta-Picolin als Stickstoffbase und führt die thermische Behandlung bei verschiedenen Temperaturen durch. Die Temperaturen sowie die hierbei erzielten Ergebnisse sind aus der nachfolgenden Tabelle zu entnehmen.

| Temperatur | %-Gehalt Beta Picolin nach thermischer Behandlung | % Rückge- winnung | % Co- Verlust |
|---|---|---|---|
| a) 185°C | 0,63 | 10,9 | 0 |
| b) 200°C | 1,20 | 23,6 | 0 |
| c) 250°C | 2,61 | 51,5 | 0 |
| d) 300°C | 2,80 | 55,3 | 30 |

Beispiel 3

Man verfährt wie in Beispiel 1 beschrieben, verwendet Beta-Picolin als Stickstoffbase und führt die thermische Behandlung bei einer Temperatur von 300°C durch mit dem Unterschied, daß das Hochdruckgefäß im kalten Zustand mit 10 bar Luft beaufschlagt wurde. Im so erhaltenen Austrag sind 3,7 Gew.-% Picolin enthalten. Die Rückgewinnungsrate beträgt 73,1 %. Cobaltabscheidung tritt nicht ein.

Beispiel 4

Man verfährt wie in Beispiel 1 beschrieben, verwendet jedoch die aus der oxidativen Entkobaltung stammende wäßrige Phase mit 2,56 Gew.-% Beta-Picolin unmittelbar für die thermische Behandlung, die bei einer Temperatur von 250°C durchgeführt wurde. Die so erhaltene wäßrige Lösung hatte einen Gehalt von 3,4 Gew.-% Beta-Picolin, was einer Rückgewinnung von 20,7 % entspricht. Cobaltabscheidung tritt nicht ein.

Beispiel 5

Man verfährt wie in Beispiel 1 beschrieben; 20,4 % des Beta-Picolins wurden umgewandelt. Man trennt restliches Beta-Picolin aus der wäßrigen Phase der oxidativen Entcobaltung durch Azeotropdestillation vollständig ab und unterwirft die verbleibende wäßrige Lösung einer Umsetzung mit $CO/H_2 = 1 : 1$ Mol/Mol bei 150°C und 280 bar. Der gebildete Cobaltcarbonylwasserstoff mit Pentan extrahiert und die verbleibende Wasserphase über 4 Std. bei 250°C unter autogenem Druck behandelt. Der Austrag enthält 2,25 % Beta-Picolin , was einer Rückgewinnung von 59,1 % entspricht.

Beispiel 6

Man verfährt wie in Beispiel 1 beschrieben (19,6 % Beta-Picolin-Umwandlung), trennt freies Beta-Picolin aus der wäßrigen Phase der oxidativen Entcobaltung durch Azeotropdestillation vollständig ab und fällt aus der verbleibenden wäßrigen Lösung das enthaltene Cobalt nach Neutralisation mit NaOH durch 10-fach molaren Überschuß an $Na_2CO_3$ als schwerlösliches $CoCO_3$. Die Mutterlauge wird über 4 Std. bei 250°C unter autogenem Druck behandelt. Der Austrag enthält 1,88 % Beta-Picolin, was einer Rückgewinnung von 51,5 % entspricht.


**Patentansprüche**

1. Verfahren zur Behandlung von wäßrigen Lösungen, die bei der Carbalkoxylierung von olefinisch ungesättigten Verbindungen anfallen und die heterocyclische aromatische tert. Stickstoffbasen, deren Umwandlungsprodukte sowie niedere Fettsäure und/oder deren Salze enthalten, dadurch gekennzeichnet, daß man
a) die wäßrige Lösung unter einem Druck von 10 bis 300 bar auf eine Temperatur von 150 bis 300 °C erhitzt und
b) anschließend heterocyclische aromatische Stickstoffbasen, die aus den Umwandlungsprodukten zurückgebildet wurden, abtrennt.
2. Verfahren nach Anspruch 1, dadurch gekennzeichnet, daß man aus der wäßrigen Lösung zunächst freie heterocyclische aromatische tert. Stickstoffbasen abtrennt.
3. Verfahren nach Anspruch 1 und 2, dadurch gekennzeichnet, daß die verwendete Ausgangslösung einen Gehalt an Kobaltsalzen mit niederen Fettsäuren hat.
4. Verfahren nach den Ansprüchen 1 bis 3, dadurch gekennzeichnet, daß man die thermische Behandlung in Stufe a unter Mitverwendung von Oxidationmitteln durchführt.
5. Verfahren nach den Ansprüchen 1 bis 4, dadurch gekennzeichnet, daß man die thermische Behandlung bei einer Temperatur von 150 bis 240°C durchführt.

6. Verfahren nach den Ansprüchen 1 bis 5, dadurch gekennzeichnet, daß man die heterocyclischen aromatischen Stickstoffbasen als Azeotrope mit Wasser abtrennt.

## Claims

1. A process for the treatment of an aqueous solution which is obtained in the carbalkoxylation of olefinically unsaturated compounds and contains heterocyclic aromatic tertiary nitrogen bases, their transformation products and lower fatty acids and/or their salts, wherein
   a) the aqueous solution is heated to 150–300°C under from 10 to 300 bar and
   b) heterocyclic aromatic nitrogen bases which are formed back from the transformation products are then separated off.
2. A process as claimed in claim 1, wherein free heterocyclic aromatic tertiary nitrogen bases are first separated off from the aqueous solution.
3. A process as claimed in claims 1 and 2, wherein the starting solution used contains cobalt salts of lower fatty acids.
4. A process as claimed in claims 1 to 3, wherein the thermal treatment in stage a is carried out in the presence of an oxidizing agent.
5. A process as claimed in claim 1 to 4, wherein the thermal treatment is carried out at from 150 to 240°C.
6. A process as claimed in claims 1 to 5, wherein the heterocyclic aromatic nitrogen bases are separated off as azeotropes with water.

## Revendications

1. Procédé pour le traitement de solutions aqueuses obtenues lors de la carbalcoxylation de composés oléfiniquement insaturés et contenant des bases azotées tertiaires aromatiques hétérocycliques, des produits de transformation de celles-ci ainsi que des acides gras inférieurs et/ou des sels de ceux-ci, caractérisé en ce que
   a) l'on chauffe la solution aqueuse sous une pression de 10 à 300 bars et à une température de 150 à 300°C et
   b) l'on sépare ensuite les bases azotées aromatiques hétérocycliques, qui se sont reformées à partir des produits de transformation.
2. Procédé suivant la revendication 1, caractérisé en ce que l'on sépare d'abord de la solution aqueuse des bases azotées tertiaires aromatiques hétérocycliques libres.
3. Procédé suivant les revendications 1 et 2, caractérisé en ce que la solution de départ utilisée contient des sels de cobalt d'acides gras inférieurs.
4. Procédé suivant les revendications 1 à 3, caractérisé en ce que l'on effectue le traitement thermique dans l'étape a) en utilisant conjointement des agents d'oxydation.
5. Procédé suivant les revendications 1 à 4, caractérisé en ce que l'on effectue le traitement thermique à une température de 150 à 240°C.
6. Procédé suivant les revendications 1 à 5, caractérisé en ce que l'on sépare les bases azotées aromatiques hétérocycliques sous forme d'azéotropes avec l'eau.